# EUROPÄISCHE PATENTSCHRIFT

(11) **EP 1 575 905 B1**
(45) Veröffentlichungstag und Bekanntmachung des Hinweises auf die Patenterteilung: **09.09.2009**
(21) Anmeldenummer: 03789263.5
(22) Anmeldetag: 13.12.2003
(51) Int. Cl.: C07C 263/10, C07C 263/20, C07C 265/14

(54) **VERFAHREN ZUR ABTRENNUNG VON ISOCYANATEN AUS EINEM REAKTIONSGEMISCH**
METHOD FOR SEPARATING ISOCYANATES OUT FROM A REACTION MIXTURE
PROCEDE DE SEPARATION D'ISOCYANATES A PARTIR D'UN MELANGE REACTIONNEL

(30) Priorität: 19.12.2002 DE 10260093
(43) Veröffentlichungstag der Anmeldung: 21.09.2005
(73) Patentinhaber: BASF SE, 67056 Ludwigshafen (DE)
(72) Erfinder: SOHN, Martin, 68229 Mannheim (DE); STROEFER, Eckhard, 68163 Mannheim (DE); NEVEJANS, Filip, B-9170 St.Gillis-Waas (BE); PENZEL, Ulrich, 01945 Tettau (DE); PALLASCH, Hans-Jürgen, 67169 Kallstadt (DE); SANDER, Michael, 67117 Limburgerhof (DE); SCHWARZ, Hans, Volkmar, B-3090 Overijse (BE); MACKENROTH, Wolfgang, 67089 Bad Dürkheim (DE)
(86) Internationale Anmeldenummer: PCT/EP2003/014186
(87) Internationale Veröffentlichungsnummer: WO 2004/056757

(56) Entgegenhaltungen:
- EP-A- 1 371 633
- WO-A-99/40059
- DE-A- 2 124 498
- US-A- 3 140 305
- US-A- 3 405 040
- US-A- 3 410 888
- US-A- 5 849 947

## Beschreibung

Die Erfindung betrifft ein Verfahren zur Abtrennung und Reinigung des Isocyanats aus einem Reaktionsgemisch in der Herstellung von Toluylendiisocyanat (TDI).

Die kontinuierliche Herstellung von organischen Isocyanaten durch Reaktion von primären organischen Aminen mit Phosgen ist vielfach beschrieben und wird im großtechnischen Maßstab durchgeführt (s. z.B. Ullmanns Enzyklopädie der Technischen Chemie, and 7 (Polyurethane), 3. neubearbeitete Auflage, Carl Hanser Verlag, München-Wien, S. 76ff (1993)). Insbesondere die aromatischen Isocyanate TDI (Toluylendiisocyanat) und MDI (Methylendiphenyldiisocyanat) bzw. PMDI (Polymethylenpolyphenylenpolyisocyanat) sowie die aliphatischen Isoyanate HDI (Hexamethylendiphenyldiisocyanat) und Isophorondiisocyanat (IPDI) werden großtechnisch hergestellt.

Die Herstellung von Isocyanaten aus den entsprechenden Aminen durch Phosgenierung erfolgte bisher zumeist in Rührkesseln, wie beispielsweise in DE-A-1468445 beschrieben, in Rührkesselkaskaden, wie beispielsweise in DE-PS 844896 beschrieben, in mit Füllkörpern gefüllten Reaktionssäulen oder Reaktionskolonnen, wie beispielsweise in WO 99/5428, und DE-A-2112181 beschrieben oder in ungefüllten Kolonnen. Oftmals ist eine Kreislauffahrweise erforderlich, um genügend Verweilzeit für einen vollständigen Umsatz bei begrenztem Reaktionsvolumen (Holdup) zu schaffen. Da die Reaktion von Amin und Phosgen in der Flüssigphase sehr schnell ist, wird für die erste Reaktionsstufe häufig ein Mischreaktor mit einer hohen Scherung des durch die Mischeinrichtung geführten Reaktionsstroms eingesetzt. Zu den bekannten Mischaggregaten gehören vor allem Düsen wie die Ringsschlitzdüse, Ringlochdüsen, Glattstrahlmischdüsen, Fächerstrahldüsen, Winkelstrahlkammerdüsen, Dreistromdüsen, Gegenstrommischkammern, Staudüsen und Venturimischdüsen.

Die Isocyanatsynthese erfolgt häufig in der ersten Stufe bei sehr tiefer und in der zweiten bei deutlich höherer Temperatur in einem Verweilzeitapparat. Dieses Verfahren wird häufig als Kalt-Heiß-Phosgenierung bezeichnet. Eine Beschreibung findet sich beispielsweise in W. Siefken, Liebigs Analen der Chemie 562 (1949), Seite 96. Zuerst wird bei tiefer Temperatur, zumeist bei 0°C oder Raumtemperatur, maximal 60°C, eine Suspension der Zwischenprodukte Carbamoylchlorid und Aminhydrochlorid hergestellt, die dann bei höheren Temperaturen, zumeist 100 bis 200°C, in einem verweilzeitapparat zum Isocyanat umgesetzt wird. Solche zweistufige Verfahren werden in Ullmanns Enzyklopädie der Technischen Chemie, Band 7 (Polyurethane), 3. neubearbeitete Auflage, Carl Hanser Verlag, München-Wien, S. 76ff (1993), und beispielsweise in den Patentschriften DE 2058032, DE 2153268 und DE 2908703 beschrieben.

Als Verweilzeitapparate können die für die Herstellung der Isocyanate üblichen und bekannten, oben beispielhaft aufgeführten Reaktoren eingesetzt werden.

Die Herstellung der Isocyanate erfolgt zumeist in Lösung. Als Lösungsmittel für die Herstellung der Isocyanate werden vorzugsweise chlorierte aromatische Kohlenwasserstoffe wie Dichlorbenzol, Chlorbenzol, Trichlorbenzol oder aromatische oder aliphatische Kohlenwasserstoffe wie Toluol, Xylol, Benzol, Pentan, Hexan, Heptan, Octan, Cyclohexan, Biphenyl, Ketone wie 2-Butanon, Methylisobutylketon, Ester wie Diethylisophtalate, Ethylacetat, Butylacetat, Nitrile wie Acetonitril, oder Sulfolan u.a. verwendet.

Nach erfolgter Reaktion wird das in der Regel leichter als das Isocyanat siedende Lösungsmittel vom Isocyanat und einem eventuellen Rückstand abgetrennt und destillativ aufgearbeitet. Im Falle von Toluylendiisocyanat (TDI) erfolgt anschließend eine destillative Abtrennung des Isocyanats vom Rückstand und eine Reindestillation des Isocyanats oder eine Reinigung durch Kristallisation. Es können außerdem weitere Trennoperationen durchgeführt werden, um im Falle von TDI, oder MDI das Isomerengemisch oder im Falle von MDI das Oligomerengemisch in einzelne Fraktionen mit unterschiedlicher Isomeren- und Oligomerenzusammensetzung zu erzeugen.

Das bei der Umsetzung von aliphatischen oder aromatischen Aminen mit Phosgen zu den entsprechenden Isocyanaten anfallende Gemisch aus Phosgen und Chlorwasserstoff, das noch mehr oder weniger große Mengen Lösungsmittel enthalten kann, wird in der Regel in den meist gasförmig anfallenden Chlorwasserstoff und ein in der Regel flüssig anfallendes. Gemisch aus Phosgen und gegebenenfalls Lösungsmittel aufgetrennt. Das Phosgen bzw. Phosgen-Lösungsmittelgemisch wird dann in die Reaktion zurückgeführt.

In US 3410888 wird ein Verfahren zur Isolierung eines aromatischen Diisocyanats aus einer Reaktionsmischung beschrieben, wobei das Isocyanat zwei Phenylkerne besitzt und die Isocyanatgruppen an Kohlenstoffatomen unterschiedlicher Phenylkerne gebunden sind. Dies betrifft 4,4'-, 2,4'-, 2,2'-Methylendi(phenylisocyanat) (MDI) sowie Mischungen dieser Isomere oder Polymethylenpolyphenylenpolyisocyanat (PMDI). Das dort beschriebene Verfahren umfaßt die Schritte erstens der Reaktion eines entsprechenden aromatischen Diamins mit Phosgen und der destillativen Abtrennung eines Teils des so hergestellten aromatischen Isocyanats im Zuge der Lösungsmittelabtrennung, zweitens der Überführung des Destillationsrückstandes (Sumpfproduktes) in eine zweite Destillationseinrichtung, die als Gefäß ausgestaltet ist, über dessen innere Oberfläche der Rückstand als dünner Film verteilt ist und dessen Temperatur und Druck ausreichend sind, um eine Verdampfung des Isocyanats zu bewirken, und drittens der Entnahme des Dampfes aus dieser zweiten Destillationseinrichtung, der im wesentlichen reich an Isocyanat ist. Der Dampf wird kondensiert und das Isocyanat gelagert. Als mögliche Destillationseinrichtung werden beispielsweise Kletterfilmverdampfer oder Fallfilmverdampfer genannt. Das gewählte Lösungsmittel in der Isocyanatsynthese hat üblicherweise einen niedrigeren Siedepunkt als das Isocyanat, er ist bevorzugt mindestens 30°C niedriger. Bei einer kleineren Siedepunktsdifferenz wird allerdings in der Lösungsmittelabtrennung ein Teil des hergestellten Isocyanats zusammen mit dem Lösungsmittel mitabgetrennt. Daran schließt sich die Destillation des als Rückstand erhaltenen Rohisocyanats im Dünnschichtverdampfer an. Die teilweise Abtrennung des Isocyanats in der Lösungsmittelabtrennung hat den Vorteil, dass unerwünschte Mittelsieder, gegebenenfalls gefärbte Verunreinigungen oder Komponenten, deren Siedepunkt zwischen dem des Isocyanats und dem Lösungsmittel liegt, in der Lösungsmittelabtrennung mit abgetrennt werden. Die Mischung aus dem teilweise abgetrennten Isocyanat und dem Lösungsmittel wird dann wieder als Einsatzstoffstrom der Lösungsmittelabtrennung zugeführt, oder sie wird in einer separaten Verdampfung oder fraktionierten Destillation zur Aufkonzentration des Isocyanats zugeführt. Letzteres wird dann als Feed in die Lösungsmittelabtrenung rezykliert.

Nachteilig an diesem Verfahren ist die teilweise Abtrennung des Isocyanats in der Lösungsmittelabtrennung, die eine zusätzliche destillative Reinigung des Lösungsmittels erforderlich macht. Enthält das Lösungsmittel zur Herstellung der Aminlösung Isocyanat, bilden sich beim Vermischen von Amin und Lösungsmittel Harnstoffe, die als Feststoffe zum einen zu Verstopfung und zum anderen zu schlechter Produktqualität führen.

In US 5,849,947 wird ein Verfahren zur Herstellung von TDI durch Umsetzung von TD1 mit Phosgen beschrieben, wobei der TDI in eine Kolonne mit Seitenabgang gereinigt wird. Daher wird aus dem Sumpf der Kolonne diskontinuierlich Produkt entnommen und einem Dünnschichtverdampfer zugeführt.

Dadurch wird das Sumpfprodukt thermisch stark belastet, was zur verstärkten Ausbildung von Nebenprodukten führt.

Bei der Herstellung von Isocyanaten im großtechnischen Maßstab in sogenannten Worldscale-Anlagen, das heißt Anlagen mit einer Kapazität von mindestens 160,000 Jahrestonnen Isocyanat, fallen große Mengen eines schwersiedenden Rückstandes an, welcher schwierig zu handhaben und teuer in der Entsorgung ist. Der Rückstand besteht zumeist aus Nebenprodukten, die durch Oligomerisierung, Polymerisation oder durch unerwünschte Neben- und Folgereaktionen gebildet werden. Durch Minimierung der thermischen Belastung und der Verweilzeit in den Destillationskolonnen, insbesondere denen zur Abtrennung des Isocyanats aus dem Reaktionsgemisch und der Reindestillation des Isocyanats, ist daher eine signifikante Verminderung des Schwersiederanfalls möglich.

Es war somit die Aufgabe der vorliegenden Erfindung, ein Verfahren bereitzustellen, bei dem insbesondere in großtechnischen Anlagen die Abtrennung des Isocyanats aus dem Reaktionsgemisch und die Reindestillation des Isocyanats unter solchen Bedingungen durchgeführt wird, daß der Schwerproduktanfall minimiert wird und das Isocyanat in guter Qualität anfällt.

Es wurde überraschenderweise ein Verfahren zur Abtrennung von Isocyanaten aus Reaktionsmischungen und Reindestillation der Isocyanate in großtechnischen Anlagen gefunden, bei welchem mit weniger Apparaten bei gleicher Reinheit des gewünschten Isocyanats ein geringerer Schwersiederanfall erreicht werden kann und das unten näher beschrieben ist.

Gegenstand der Erfindung ist somit ein Verfahren zur Herstellung von Toluylendiisocyanat durch Umsetzung von Aminen mit Phosgen in einem Reaktor, gegebenenfalls Abtrennung des verwendeten Lösungsmittels und der nachfolgenden Abtrennung und Reinigung des Isocyanats aus der Reaktionsmischung, **dadurch gekennzeichnet, daß** die Abtrennung und Reinigung des Isocyanats in einer mit einer vertikalen Trennwand ausgestatteten Kolonne bei einem Kopfdruck von 5-50 mbar, besonders bevorzugt 10-20 mbar, und einer Sumpftemperatur von 90-250°C, bevorzugt 120-170°C, besonders bevorzugt 130-150°C, durchgeführt wird, und die Kolonne im Gegenstrom von Gas und Flüssigkeit betrieben wird. Die Verweilzeit im Sumpf ist nicht größer als sechs Stunden. Der Rein-Isocyanat-Strom wird flüssig oder gasförmig in einem Seitenabzug der Kolonne abgenommen.

Die Zuführung des Reaktionsgemisches erfolgt vorteilhaft im unteren Teil der Kolonne, bevorzugt kann die Kolonne auch nur mit einem reinen Verstärkungsteil ohne Abtriebsteil ausgestattet sein. Als Einbauten kommen die bekannten Einbauten von Destillations- und Rektifikationskolonnen zum Einsatz. Es kann u.a. eine Boden- oder eine Packungskolonne zum Einsatz kommen. Als Böden können beispielsweise Sieb-, Ventil-, Glocken- oder Dualflowböden und bei Packungen z.B. Blech-, Gewebe- oder Gitterpackungen aller Art verwendet werden. Besonders vorteilhaft sind Packungen, da sie einen geringen Druckverlust aufweisen. Füllkörperschüttungen sind jedoch weniger geeignet, aber nicht prinzipiell ausgeschlossen. Als Packungstypen können beispielsweise Sulzer BX, Sulzer CY, Sulzer Mellapak, Sulzer Mellapak Plus, Montz A3, Glitsch 4A, Kühni Rombopak, und andere. verwendet werden. Als Sumpfumlaufverdampfer können prinzipiell alle Arten von Verdampfertypen eingesetzt werden, wobei Fallfilmverdampfer, Langrohrverdampfer oder Dünnschichtverdampfer besonders vorteilhaft sind, da sich mit ihnen eine produktschonende Verdampfung realisieren läßt. Aus energetischen Gründen und um das Produkt zu schonen und so den Schwerproduktanfall zu minimieren, kann es vorteilhaft sein, der erfindungsgemäß verwendeten Kolonne eine ein- oder mehrstufige Verdampfung vorzuschalten. Auch eine Zwischenverdampfung ist vorteilhaft. Bei der Vorverdampfung wird der Flüssigkeitszulauf einem Verdampfer zugeführt und dadurch teilweise oder ganz verdampft. Der Dampf- und gegebenenfalls der verbliebene Flüssigkeitsstrom wird der Kolonne zugeführt. Bei einer Zwischenverdampfung wird die.Flüssigkeit entsprechend von einem Boden oder Sammler der Kolonne entnommen und einem Wärmetauscher zugeführt. Sowohl die Vorverdampfung als auch die Zwischenverdampfung können ein- oder mehrstufig ausgeführt werden. Der Kopfkondensator kann extern gestaltet oder in die Kolonne integriert sein. Es können Rohrbündel- wie auch Plattenapparate zum Einsatz kommen.

Prinzipiell kann das verwendete Lösungsmittel noch in der Reaktionsmischung, die in die erfindungsgemäß verwendete Kolonne enthalten sein. Es ist jedoch vorteilhaft, zumindest einen Teil davon vorher abzutrennen. Dies kann beispielsweise in einer vorgeschalteten Kolonne oder einer ähnlichen Trenneinrichtung erfolgen.

Am Kopf der Kolonne fallen u.a. Chlorwasserstoff, Phosgen, Lösungsmittel, chlorierte Nebenprodukte und Inerte wie Stickstoff und Kohlendioxid an.

Das am Sumpfaustrag der Kolonne abgezogene Schwerprodukt enthält hochsiedende oligomere und polymere Verbindungen, typischerweise zumeist Harnstoffe, Polyharnstoffe, Isocyanurate, Uretdione, Carbodiimide sowie nicht vollständig abgetrenntes Isocyanat.

Wenn das ausgeschleuste Sumpfprodukt der Kolonne noch Isocyanat enthält, kann dieses vorteilhaft zur Gewinnung des im Rückstand noch verbliebenen Isocyanats in einem weiteren Apparat, bevorzugt einer Kolonne, bei einem Druck von 1-500 mbar, bevorzugt 5-25 mbar, und einer Temperatur von 100-225°C, bevorzugt 110-140°C, bis auf eine Konzentration von < 10 Gew.-% bezüglich des Feedstroms der ersten Kolonnen abgereichert werden. Der Sumpfaustrag dieser Kolonne kann noch einmal aufgearbeitet werden, um weiteres Rest-Isocyanat aus dem Schwerprodukt zu gewinnen. Alle so erhaltenen Isocyanatfraktionen können der ersten Kolonne zur Reinigung des Isocyanats wieder zugeführt werden.

Das erfindungsgemäße Verfahren eignet sich für die Aufarbeitung von Toluylendiisocyanat (TDI).

TDI neigt zur Ausbildung von Schwerprodukten, die sehr schwierig zu handhaben sind und die unter Umständen die Verfügbarkeit von TDI-Anlagen mindern. Durch die erfindungsgemäße Ausgestaltung der Parameter der Kolonne zur Abtrennung des TDI kann die Ausbildung der Feststoffe merklich zurückgedrängt werden. Dieser Effekt macht sich besonders bei großtechnischen Anlagen mit einer Kapazität von mindestens 160,000 Jahrestonnen bemerkbar.

Die Erfindung soll an dem nachstehenden Referenzbeispiel näher erläutert werden.

### Referenzbeispiel:

Von einem Reaktionsaustrags aus der Synthese von Toluylendiisocyanat (TDI) ausgehend von Toluylendiamin (TDA) und Phosgen, von dem das Lösungsmittel abgetrennt worden ist, wurde dem unteren Teil einer Destillationskolonne mit 50 mm Durchmesser zugeführt. Die Kolonne war mit 12 Schüssen Gitterpackung (Kühni Rombopak 9M,

Länge eines Schusses 630 mm) gepackt. Die Sumpftemperatur betrug 145°C und der Kopfdruck 15 mbar abs. Als Verdampfer wurde ein Dünnschichtverdampfer eingesetzt. Die Zusammensetzung des zulaufs (1,14 kg/h) war 1,1 kg/h (96,5 Gew.-%) TDI inklusive schwersiedender TDI-Homologen, 0,02 kg/h (1,8 Gew.-%) Uretdion und 0,02 kg/h (1,8 Gew.-%) chlorierte Nebenprodukte sowie geringe Mengen Leichtsieder wie Chlorwasserstoff, Phosgen, und andere. An einem Seitenabzug der Kolonne wurden 1,0 kg/h (99,9 Gew.-%) TDI mit geringen Mengen (0,001 kg/h, 0,1 Gew.-%) chlorierter Nebenprodukte entnommen. Am Kopf der Kolonne wurden hinter dem Kopfkondensator, einem Rohrbündelapparat mit 13 Rohren, 0,018 kg/h Leichtsieder. vorwiegend Chlorwasserstoff und Phosgen, gasförmig entnommen und einer alkalischen Wäsche zur Vernichtung zugeführt. Das im Wärmetauscher anfallende Kondensat des Brüden wurde als Rücklauf auf den Kopf der Kolonne gegeben. Am Sumpf der Kolonne wurden 0,12 kg/h entnommen und einer einstufigen Verdampfung, die bei 5 mbar und 115°C betrieben wurde, zugeführt. Es wurden 0,06 kg/h TDI dampfförmig entnommen, kondensiert und mit dem anderen, am Seitenabzug der ersten Kolonne gewonnen TDI, vereinigt. Der zurückbleibende teerartige Rückstand wurde der Verbrennung zugeleitet.

## Patentansprüche

1. Verfahren zur Herstellung von Toluylendiisocyanat durch Umsetzung von Aminen mit Phosgen in einem Reaktor und die nachfolgende Abtrennung und Reinigung des Isocyanats aus der Reaktionsmischung, **dadurch gekennzeichnet, daß** die Abtrennung und Reinigung des Isocyanats in einer Kolonne bei einem Kopfdruck von 5-50 mbar, bevorzugt 10-20 mbar, und einer Sumpftemperatur von 90-250°C, bevorzugt 120-170°C, besonders bevorzugt 130-150°C, durchgeführt wird, und die Kolonne im Gegenstrom von Gas und Flüssigkeit betrieben wird, wobei der Rein-Isocyanat-Strom in einem Seitenabzug der Kolonne flüssig oder gasförmig abgenommen wird und die Verweilzeit im Sumpf der Kolonne nicht größer als 6 Stunden, bezogen auf den Sumpfabzug, ist, und dass die Kolonne eine vertikale Tennwand aufweist.

2. Verfahren nach Anspruch 1, **dadurch gekennzeichnet, dass** das Sumpfprodukt der Kolonne noch Isocyanat enthält, welches in einem weiteren Apparat bei einem Druck von 1-500 mbar, bevorzugt 5-25 mbar, und einer Temperatur von 100-225°C, bevorzugt 110-140°C, bis auf eine Konzentration von < 10 Gew.-% bezüglich des Feedstroms der ersten Kolonne, abgereichert wird.

3. Verfahren nach einem der Ansprüche 1 bis 2, **dadurch gekennzeichnet, dass** der Kolonne eine ein- oder mehrstufige Verdampfung vorgeschaltet ist.

4. Verfahren nach einem der Ansprüche 1 bis 3, **dadurch gekennzeichnet, dass** eine Zwischenverdampfung an der Kolonne durchgeführt wird.

5. Verfahren nach einem der Ansprüche 1 bis 4, **dadurch gekennzeichnet, dass** als Verdampfer für die Kolonne, die Vorverdampfung und die Zwischenverdampfung ein Durchlaufverdampfer, vorzugsweise ein Fallfilmverdampfer, Langrohrverdampfer oder Dünnschichtverdampfer verwendet wird.

6. Verfahren nach einem der Ansprüche 1 bis 5, **dadurch gekennzeichnet, dass** die Kolonne mit einer Blech-, Gewebe- oder Gitterpackung gepackt ist.

7. Verfahren nach einem der Ansprüche 1 bis 6, **dadurch gekennzeichnet, dass** die Verweilzeit im Sumpf der Kolonne nicht größer als vier Stunden, bezogen auf den Sumpfabzug, ist.

8. Verfahren nach einem der Ansprüche 1 bis 7, **dadurch gekennzeichnet, dass** die Reaktionsmischung im unteren Teil der ersten Kolonne zur Abtrennung des Isocyanats zugeführt wird.

## Claims

1. A process for preparing tolylene diisocyanate by reaction of amines with phosgene in a reactor and subsequent separation of the isocyanate from the reaction mixture and purification of the isocyanate, wherein the separation and purification of the isocyanate is carried out in a column having a pressure at the top of 5-50 mbar, preferably 10-20 mbar, and a temperature at the bottom of 90-250°C, preferably 120-170°C, particularly preferably 130-150°C, and the column is operated with countercurrent flow of gas and liquid, the pure isocyanate stream being taken off in liquid or gaseous form at a side offtake of the column and the residence time in the bottom of the column being not more than 6 hours, based on the product taken off at the bottom, and wherein the column has a vertical dividing wall.

2. The process according to claim 1, wherein the bottom product from the column still comprises isocyanate which is depleted in a further apparatus at a pressure of 1-500 mbar, preferably 5-25 mbar, and a temperature of 100-225°C, preferably 110-140°C, down to a concentration of <10% by weight based on the feed stream to the first column.

3. The process according to either of claims 1 and 2, wherein the column is preceded by a single-stage or multistage vaporization.

4. The process according to any of claims 1 to 3, wherein an intermediate vaporization is carried out on the column.

5. The process according to any of claims 1 to 4, wherein a flow-through vaporizer, preferably a falling film evaporator, long tube evaporator or thin film evaporator, is used as vaporizer for the column, the preliminary vaporization and the intermediate vaporization.

6. The process according to any of claims 1 to 5, wherein the column is packed with sheet metal packing, woven fabric packing or mesh packing.

7. The process according to any of claims 1 to 6, wherein the residence time in the bottom of the column is not more than four hours, based on the product taken off at the bottom.

8. The process according to any of claims 1 to 7, wherein the reaction mixture is fed into the lower part of the first column for separating off the isocyanate.

## Revendications

1. Procédé de fabrication de diisocyanate de toluylène par réaction d'amines avec du phosgène dans un réacteur et séparation et purification ultérieures de l'isocyanate à partir du mélange réactionnel, **caractérisé en ce que** la séparation et la purification de l'isocyanate sont réalisées dans une colonne à une pression de tête de 5 à 50 mbars, de préférence de 10 à 20 mbars, et à une température de fond de 90 à 250 °C, de préférence de 120 à 170 °C, de manière particulièrement préférée de 130 à 150 °C, et la colonne est utilisée en présence de gaz et de liquide, le courant d'isocyanate pur étant extrait sous forme liquide ou gazeuse au niveau d'une sortie latérale de la colonne et le temps de séjour au fond de la colonne n'étant pas supérieur à 6 heures par rapport à la sortie de fond, et **en ce que** la colonne comporte une paroi de séparation verticale.

2. Procédé selon la revendication 1, **caractérisé en ce que** le produit de fond de la colonne contient encore de l'isocyanate, qui est appauvri dans un autre appareil à une pression de 1 à 500 mbars, de préférence de 5 à 25 mbars, et à une température de 100 à 225 °C, de préférence de 110 à 140 °C, jusqu'à une concentration < 10 % en poids par rapport au courant d'alimentation de la première colonne.

3. Procédé selon l'une quelconque des revendications 1 à 2, **caractérisé en ce que** la colonne est connectée en amont à une évaporation à une ou plusieurs étapes.

4. Procédé selon l'une quelconque des revendications 1 à 3, **caractérisé en ce qu'**une évaporation intermédiaire est réalisée dans la colonne.

5. Procédé selon l'une quelconque des revendications 1 à 4, **caractérisé en ce qu'**en tant qu'évaporateur pour la colonne, la pré-évaporation et l'évaporation intermédiaire, un évaporateur à écoulement continu, de préférence un évaporateur à film tombant, un évaporateur à tube long ou un évaporateur à couche mince est utilisé.

6. Procédé selon l'une quelconque des revendications 1 à 5, **caractérisé en ce que** la colonne est garnie avec un garnissage en tôle, en tissu ou en grille.

7. Procédé selon l'une quelconque des revendications 1 à 6, **caractérisé en ce que** le temps de séjour dans le fond de la colonne n'est pas supérieur à quatre heures, par rapport à la sortie de fond.

8. Procédé selon l'une quelconque des revendications 1 à 7, **caractérisé en ce que** le mélange réactionnel est introduit dans la partie inférieure de la première colonne pour la séparation de l'isocyanate.
